# EUROPEAN PATENT SPECIFICATION

(11) **EP 1 768 673 B1**
(45) Date of publication and mention of the grant of the patent: **30.07.2008**
(21) Application number: 05757277.8
(22) Date of filing: 14.06.2005
(51) Int. Cl.: A61K 31/519, A61K 9/00

(54) **ORALLY DISINTEGRATING PHARMACEUTICAL COMPOSITION COMPRISING RISPERIDONE**
IM MUND ZERFALLENDE PHARMAZEUTISCHE ZUSAMMENSETZUNG MIT RISPERIDON
COMPOSITION PHARMACEUTIQUE A BASE DE RISPERIDONE SE DESINTEGRANT ORALEMENT

(30) Priority: 15.06.2004 DE 102004028940
(43) Date of publication of application: 04.04.2007
(73) Proprietor: KRKA, tovarna zdravil, d.d., Novo mesto, 8501 Novo mesto (SI)
(72) Inventor: KROSELJ, Vesna;, 8310 Sentjernej (SI); RAJER, Tadeja, 8000 Novo mesto (SI); RANGUS, Marija;, 8310 Sentjernej (SI); GUSTIN, Jozica, 8000 Novo mesto (SI)
(74) Representative: UEXKÜLL & STOLBERG
(86) International application number: PCT/EP2005/006375
(87) International publication number: WO 2005/123084

(56) References cited:
- EP-A1- 1 413 294
- WO-A-97/48383
- WO-A-20/04091585
- WO-A-20/05120463

## Description

The present invention relates to an orally disintegrating pharmaceutical composition which comprises risperidone and a process for its preparation.

Risperidone is known to be useful in the treatment of psychotic diseases and has been disclosed in EP 368 388. It is represented by the following structural formula:

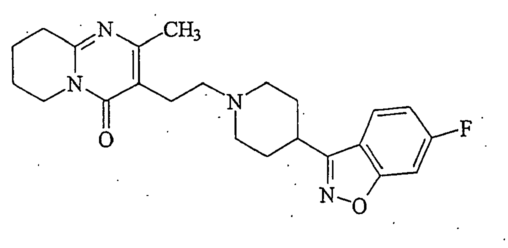

In the field of pharmacy, there have been in the past attempts to provide a type pharmaceutical composition for oral administration which results in improved patient compliance in comparison to conventional solid dosage forms such as capsules and tablets. In particular pediatric and geriatric patients often experience difficulties in swallowing solid dosage forms. Besides, conventional solid dosage forms are not suitable in case the patient has no easy access to water. Thus, orally disintegrating compositions represent an alternative for such patients.

A satisfactory orally disintegrating dosage form needs to meet a number of requirements. Firstly, it has to disintegrate in the mouth spontaneously. However, the active ingredient should not be released from the dosage form in the oral cavity as is the case for sublingual or buccal formulations. Otherwise the active ingredient would be absorbed already in the mouth leading to a different distribution and metabolization of the ingredient in the body compared to a conventional tablet which releases the active ingredient in the gastro-intestinal tract. Moreover, a premature release in the mouth could also lead to problems due to the often unpleasant taste of the active ingredient.

To fulfil all these requirements the formulation for a specific drug needs to be adapted in particular by a careful selection of the excipients used. However, the excipients may cause a problem as they may lead to formulations which are not bioequivalent to the corresponding conventional dosage forms.

Orally disintegrating pharmaceutical compositions are known.

For example orally disintegrating tablets which contain risperidone as active ingredient are sold under the tradename Risperdal^{®} Quicklet^{®}. They are prepared by a process which is described in EP-B-910 345. This process allows the preparation of solid rapidly disintegrating dosage forms which have the form of biconvex tablets. The process comprises the overfilling of' a mold with a predetermined amount of an aqueous composition, and freezing and removing the solvent from the frozen composition by lyophilization.

A significant drawback of this very specific process is that it leads to high production costs, in particular due to the use of the long duration of each freeze drying cycle, normally 24 to 48 h and the necessity to use sophisticated equipment for carrying out the process.

WO 2004/022037 discloses taste masked dosage forms comprising drugs of a great variety of different therapeutic categories and one or more cationic polymers prepared from dimethylaminoethyl methacrylate and neutral methacrylic acid esters, wherein the wt/wt ratio of the drug to the polymer is less than about 1 to 2. These dosage forms are prepared by loading of a solution or dispersion of the drug onto an inert core and coating it with one or more cationic polymers.

It is a disadvantage of this process that it requires a curing of the coating layer for 24 hours at room temperature. Moreover, the process requires use of an inert core the presence of which may lead to segregation problems when compressing the coated particles to produce tablets. Thus, the inert core includes compressible excipients which are far more expensive than conventional excipients. Finally, the coating composition usually includes organic solvents which are undesirable due to environmental and health concerns, and may lead to undesired residual solvents in the final product.

WO 03/103629 describes orally disintegrating tablets containing specific amounts of active ingredient having a specific particle size distribution, spray-dried mannitol, microcrystalline cellulose of a specific average particle size and a specific particle size distribution, and sodium croscarmellose. The tablets are prepared by a direct compression process and therefore specials excipients are required. In particular a diluent is used which shows a high compressibility like spray-dried mannitol.

WO 2004/091585 is directed to orally disintegrating tablets comprising an effective amount of an active agent and an amount of silicified microcrystalline cellulose. EP-A-1 413 294 describes compositions containing sustained-release fine grains for tablets which are quickly disintegrable in the oral cavity. In particular, the compositions comprise the product granulation of sustained-release particles containing a drug and one or two or more fillers selected from sugars or sugar alcohols.

Finally, EP-A-1 323 417 discloses tablets which are rapidly disintegrating in the oral cavity and comprise a drug, diluent and a saccharide which has melting point below that of the drug and the diluent. The tablets are prepared by melting and subsequently solidifying the saccharide whereupon bridges are formed between the drug and the diluent. However, the required heating step to melt the saccharine requires energy and may lead to degradation of the components of the tablet and in particular of the drug. Such a degradation is generally highly undesirable in case of tablets for human use.

Thus, there is a need for an orally disintegrating composition of risperidone which avoids the above mentioned problems and in particular disintegrates quickly in the oral cavity without releasing substantial amounts of the active ingredient and hence avoids the occurrence of the extremely bitter taste of risperidone. Moreover, such a composition should not requires the use of expensive excipients and should be obtainable by a simple and quick process which does not need the use of specific equipment or of organic solvents.

These problems are surprisingly solved by the orally disintegrating pharmaceutical composition according to claims 1 to 14. The invention also relates to the process according to claims 15 and 16.

The orally disintegrating pharmaceutical composition according to the invention comprises
(a) risperidone or a salt or solvate thereof,
(b) polymethacrylate,
(c) sugar alcohol, and
(d) disintegrant comprising (i) cross-linked polyvinyl pyrrolidone and (ii) low-substituted hydroxypropyl cellulose.

It has unexpectedly been found out that the specific combination of the excipients (b), (c) with the combination of the disintegrants (d) (i) and (d) (ii) provide a composition which allows the preparation of a composition of the drug risperidone which avoids the afore-mentioned disadvantages of the compositions of the prior art. In particular, the composition according to the invention does not require use of expensive excipients and can be prepared by a simple procedure in an efficient manner.

The component (a) of the composition according to the invention is risperidone. The risperidone can also be present in form of a salt thereof. Examples of useful salts are the addition salts with an inorganic acid selected from hydrochloric, hydrobromic, sulfuric, nitric, and phosphoric acid; or with an organic acid selected from acetic, propanoic, hydroxyacetic, lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, p-aminosalicylic, and pamoic acid. Examples of useful salts are also described in EP 368 388.

In addition, the risperidone can also be used in form of a solvate thereof with for example water or organic solvents.

Finally, the risperidone can also be used in different polymorphic forms, for example polymorphic form A as disclosed in Acta Cyst. (1993), C49, 1698-1700 or polymorphic forms A, B and E as described in WO 02/12200 and WO 02/14286 as well as polymorphic form T1 disclosed in WO 2004/020439.

It is preferred that the composition comprises 0.05 to 4.0, more preferably 0.2 to 3.0 and most preferably 0.5 to 2.0 % by weight of risperidone or a salt or solvate thereof.

It is also preferred that a single dosage form of the composition according to the invention, such as a tablet or capsule, comprises 0.25 to 16 mg risperidone or salt or solvate thereof, calculated as risperidone.

The component (b) of the composition is polymethacrylate. Polymethacrylate is a cationic, anionic or neutral polymer or copolymer on the basis of monomers having a methacrylic moiety. Mixtures of such polymers or copolymers can also be used.

Preferably, the polymethacrylate is a polymer or copolymer based on at least one of dimethylaminoethyl methacrylates, methacrylic acid and methacrylic acid esters.

It is particularly preferred that the polymethacrylate is a methacrylic acid copolymer or a copolymer of dimethylaminoethyl methacrylates and methacrylic acid esters.

Various different types of polymethacrylates are commercially available e.g. under the trade name Eudragit®. A preferred material for use in the composition according to the invention is Eudargit L which is a methacrylic acid copolymer or Eudragit EPO. Particularly preferred is Eudragit E PO which is a cationic copolymer of dimethylaminoethyl methacrylate and neutral methacrylic esters having an average molecular weight of about 150.000.

It is preferred that the composition comprises 0.1 to 20.0, more preferably 0.5 to 15.0 and most preferably 1.0 to 8.0 % by weight of polymethacrylate. Further it has proved to be possible to use relatively large amounts of polymethacrylate relative to component (a), without detrimental effect to the disintegration behavior of the composition according to the invention. The weight ratio of polymethacrylate (b) to component (a) can be at least 3:1; preferably at least 5:1.

The component (c) in the composition is a sugar alcohol. Mixtures of sugar alcohols are also operable. Preferred such sugar alcohols are sorbitol and mannitol with mannitol being particularly preferred. Mannitol is available in various forms, such as spray-dried mannitol, mannitol for direct compression, conventional mannitol and others. All these forms are operable and preferably conventional mannitol is used.

The particle size of the sugar alcohol can be in the range of 10 to 500µm. The average particle size is preferably 100 to 200µm.

It is preferred that the composition comprises 30.0 to 90.0, more preferably 40.0 to 80.0, even more preferably 50.0 to 70.0 % are most preferably 55.0 to 65.0% by weight of sugar alcohol.

The component (d) of the composition is a disintegrant which comprises (i) cross-linked polyvinyl pyrrolidone and (ii) low-substituted hydroxypropyl cellulose.

Cross-linked polyvinyl pyrrolidone is commercially available under the tradename Crospovidone.

Examples of useful low-substituted hydroxypropyl celluloses are available under the trade names LH-11, LH-20, LH-21, LH-22, LH-30, LH-31 and LH-32 from Shin-Etsu Chemical Co., Ltd.

The low-substituted hydroxypropyl cellulose has preferably a content of hydroxypropyl groups of 7.0 to 12.9 % by weight, in particular 10.0 to 12.9 % by weight.

In a further preferred embodiment the disintegrant (d) also comprises at least one additional disintegrant selected from (iii) calcium silicate, sodium starch glycolate or croscarmellose sodium. Preferably calcium silicate is chosen as additional disintegrant.

It is preferred that the composition comprises 5.0 to 50.0, more preferably 10.0 to 40.0, even more preferably 20.0 to 35.0 % and most preferably 20 to 25 % by weight of disintegrant (d).

Further, it is particularly preferred that the composition comprises 1.0 to 30.0 and in particular 5.0 to 20.0 %, most preferably 5.0 to 10.0, by weight of (d)(i) cross-linked polyvinyl pyrrolidone.

It is also particularly preferred that the composition comprises 1.0 to 20.0 and in particular 3.0 to 15.0 %, most preferably 4.0 to 9.0 by weight of (d)(ii) low-substituted hydroxypropyl cellulose.

It is also particularly preferred that the composition comprises 10 to 15 % by weight of (d) (iii) calcium silicate.

The composition according to the invention can be in any suitable form such as a tablet, a capsule or a granulate. Preferably it takes the form of a tablet.

It has surprisingly been shown that the composition according to the invention overcomes the problems associated with the conventional risperidone formulations. The composition not only disintegrate very quickly in the oral cavity, but also does not release risperidone in the mouth to an extent that its bitter taste will be perceived by a patient. Thus, the composition results in a high patient compliance.

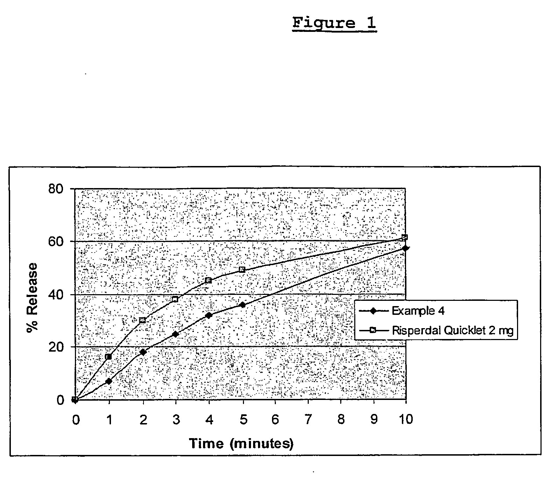

The improved prevention of release of risperidone at this undesired location is shown by an *in vitro* drug release test. This test, which is explained in detail in the examples and which results are represented in Figure 1, showed that at a pH prevailing in the oral cavity the release of risperidone from the composition according to the invention was substantially lower than that from tablets prepared according to EP-B-910 345 and which are commercially available under the trade name Risperdal^{®} Quicklet^{®}. Nevertheless, there was immediate release from the composition at the acidic pH of the gastric juices.

Moreover, the composition according to the invention can surprisingly be prepared in a very simple and economic manner. The process for preparing the composition comprises
(i) preparing granules containing risperidone or a salt or solvate thereof (a) and polymethacrylate (b), and
(ii) mixing the granules with sugar alcohol (c) and disintegrant (d) and
(iii) optionally molding the obtained mixture to the desired form.

In step (i) granules are prepared and this can be effected using mixing and granulating techniques customary in pharmacy, such as high shear granulation or fluidized bed granulation. Preferably, the granulation step is carried out by
- Mixing risperidone and polymethacrylate,
- Spraying of water on the mixture,
- Kneading of the moistened mixture,
- Drying of product of the mixture,
- Sieving of the dried granules obtained and optionally milling.

It is possible to add during this granulation step, in particular during the mixing or spraying step, also filler such as lactose and starch, and/or binder, such as gelatine, polyvinyl pyrrolidone, starch, pregelatinized starch, or cellulose derivatives, such as hydroxypropylmethyl cellulose (HPMC), hydroxypropyl cellulose (HPC) and carboxymethyl cellulose (CMC) or their salts. The preferred binder is polyvinyl pyrrolidone.

Further, it has proved particularly advantageous if the granules prepared in step (i) also contain a part of the sugar alcohol (c). In such a case the selected sugar alcohol is added at any suitable stage during the granulation step.

In step (ii) the granules are mixed with sugar alcohol (c) and disintegrants (d). In this step also additional conventional excipients may be added, such as fillers, diluents, binders, lubricants, sweeteners, flavouring agents, glidants, colouring agents, and the like.

In another embodiment, sugar alcohol (c), disintegrant (d) and optional conventional excipients can be granulated and the resulting granules be mixed with the granules from step (i).

Examples of suitable fillers and diluents are sucrose, lactose and starch.

Examples of suitable binders are gelatine, polyvinyl pyrrolidone (PVP), starch, pregelatinized starch, cellulose derivatives, such as hydroxypropylmethyl cellulose (HPMC), hydroxypropyl cellulose (HPC) and carboxymethyl cellulose (CMC) and their salts. Preferably microcrystalline cellulose is used as binder.

Examples of suitable lubricants are magnesium stearate, magnesium lauryl sulfate, sodium stearyl fumarate, fatty acid, polyethylene glycol and stearic acid.

Examples of sweeteners are acesulfame K, sucralose, alitame, aspartame, saccharin sodium, dipotassium glycyrrhizinate, stevia, and thaumatin.

Flavourings agents can be natural or synthetic materials, such as Spearmint flavour.

Glidants are preferably selected from the group consisting of silicon dioxide, talc and aluminium silicate.

Suitable colouring agents include ferric oxides.

In optional step (iii) the obtained mixture of the granules, sugar alcohol (c) and disintegrant (d) as well as optional additional excipients is molded to the desired form. This can be effected by conventional processes. In particular, tablets can be prepared by compressing the mixture.

Thus, the orally-disintegrating composition according to the invention can be prepared by a simple granulation process which usually is employed for the production of e.g. conventional tablets. In contrast to the prior art compositions, the manufacturing process is not only very simple, but also does not require use of organic solvents, expensive excipients and inert cores. Nevertheless, the compositions show the desired combination of fast disintegration in the mouth of a patient without occurrence of the bitter taste of risperidone and immediate relase of risperidone in the gastro-intestinal tract.

The invention is now explained in more detail with reference to examples.

### Examples

### Example 1 - Preparation of granules

| | |
|---|---|
| Risperidone | 100 g |
| Eudragit E PO | 500 g |
| Povidone K25 | 18 g |
| Purified water | q.s. |

| | |
|---|---|
| "q.s" means "as needed" | |

Risperidone, Polymethacrylate (Eudragit E PO) and polyvinyl pyrrolidone (Povidone K25) in the above given amounts were homogenised for 1 minute in a high shear mixer. Purified water was sprayed onto the powder mixture and the obtained moistened mixture was further kneaded for 30 seconds. The kneaded mixture was dried in a fluid bed drier and sieved to give a granulate.

### Example 2 - Preparation of granules

| | |
|---|---|
| Risperidone | 100 g |
| Eudragit E PO | 300 g |
| Mannitol | 300 g |
| Povidone K25 | 20 g |
| Purified water | q.s. |

Risperidone, Polymethacrylate (Eudragit E PO), sugar alcohol, namely mannitol, and polyvinyl pyrrolidone (Povidone K25) were homogenised for 1 minute in a high shear mixer. Purified water was sprayed onto the powder mixture and the obtained moistened mixture was further, kneaded for 30 seconds. The kneaded mixture was dried in a fluid bed drier and sieved to give a granulate

### Example 3 - Preparation of granules

| | |
|---|---|
| Risperidone | 100 g |
| Eudragit L 100 | 290 g |
| Purified water | 250 g |

Risperidone and polymethacrylate (Eudragit L 100) were homogenised for 1 minute in a high shear mixer. Purified water was sprayed onto the powder mixture and the obtained moistened mixture was further kneaded for 30 seconds. The obtained kneaded mixture was dried in a fluid bed drier and sieved to give a granulate.

### Example 4 - Preparation of orally-disintegrating tablets

| | |
|---|---|
| Granules from Example 1 | 216 g |
| Mannitol | 1578 g |
| Microcrystalline cellulose | 240 g |
| Hydroxypropyl cellulose, low substituted | 180 g |
| Aspartame | 24 g |
| Crospovidone | 240 g |
| Spearmint flavour | 12 g |
| Calcium silicate | 450 g |
| Magnesium stearate | 4 mg |
| Purified water | q.s. |

Mannitol, microcrystalline cellulose, low-substituted hydroxypropyl cellulose, cross-linked polyvinyl pyrrolidone (crospovidone) and aspartame were mixed in a high shear mixer and granulated with purified water. The obtained granulate was mixed with the granules from Example 1. Spearmint flavour and calcium silicate were added to the resulting mixture. In the end magnesium stearate was admixed and the obtained mixture was compressed to tablets at <60 N. The disintegration times of the obtained tablets were in the range of 9 to 21 s. They were measured in purified water at 37°C according to the method described in Ph. Eur. chapter 2.9.1. Test A, page 4683, supplement 4.8 07/2004.

### Examples 5 and 6 - Preparation of orally-disintegrating tablets

In the same manner as described in example 4 orally-disintegrating tablets were produced with the exception that the granules of example 1 were replaced by the granules of examples 2 and 3.

### Example 7 - Release of risperidone from orally-disintegrating tablets

In this example the release profile of risperidone from tablets according to example 4 was compared with that of the commercially available orally-disintegrating tablets Risperdal® Quicklets®.

For this purpose an *in vitro* drug-release test was carried out with either type of tablets utilizing the United States Pharmacopea (USP) paddle method with Apparatus 2 at 50 rpm with 500 mL of phosphate buffer at a pH of 6.8 which simulates the pH prevailing in the mouth.

The results of this test are shown in Fig. 1 as % by weight of risperidone release within a given period of time. It can be seen that the release of risperidone from the commercial tablets is significantly faster than from the tablets according to the invention.

## Claims

1. An orally disintegrating pharmaceutical composition which comprises
(a) risperidone or a salt or solvate thereof,
(b) polymethacrylate,
(c) sugar alcohol, and
(d) disintegrant comprising (i) cross-linked polyvinyl pyrrolidone and (ii) low-substituted hydroxypropyl cellulose.

2. Composition according to claim 1, wherein the polymethacrylate (b) is a polymer or copolymer based on at least one of dimethylaminoethyl methacrylates, methacrylic acid and methacrylic acid esters.

3. Composition according to claim 1 or 2, wherein the polymethacrylate (b) is a methacrylic acid copolymer or a copolymer of dimethylaminoethyl methacrylates and methacrylic acid esters.

4. Composition according to any one of claims 1 to 3, wherein the sugar alcohol (c) is selected from sorbitol or mannitol.

5. Composition according to any one of claims 1 to 4, wherein the low-substituted hydroxypropyl cellulose has a content .of hydroxypropyl groups of 7.0 to 12.9 % by weight.

6. Composition according to any one of claims 1 to 5, wherein the disintegrant (d) also comprises additional disintegrant selected from (iii) calcium silicate, sodium starch glycolate or croscarmellose sodium.

7. Composition according to any one of claims 1 to 6, which comprises
(a) 0.05 to 4.0 % by weight of risperidone or a salt or solvate thereof,

8. Composition according to any one of claims 1 to 7, which comprises
(b) 0.1 to 20.0 % by weight of polymethacrylate.

9. Composition according to any one of claims 1 to 8, which comprises
(c) 30.0 to 90.0 % by weight of sugar alcohol.

10. Compositon according to any one of claims 1 to 9, which comprises
(d) 5.0 to 50.0 % by weight of disintegrant.

11. Composition according to any one of claims 1 to 10, which comprises
(d)(i) 1.0 to 30.0 % by weight of cross-linked polyvinyl pyrrolidone.

12. Composition according to any one of claims 1 to 11, which comprises
(d)(ii) 1.0 to 20.0 % by weight of low-substituted hydroxypropyl cellulose.

13. Composition according to any one of claims 1 to 12, which is in form of a tablet.

14. Composition according to any one of claims 1 to 13, wherein the weight ratio of (b) to (a) is at least 3 to 1.

15. Process for preparing the' composition according to any one of claims 1 to 14 comprising
(i) preparing granules containing risperidone or a salt or solvate thereof (a) and polymethacrylate (b), and
(ii) mixing the granules with sugar alcohol (c) and disintegrant (d) and
(iii) optionally molding the obtained mixture to the desired form.

16. Process according to claim 15, wherein in step (i) the granules also contain a part of the sugar alcohol (c).

## Patentansprüche

1. Oral zerfallende pharmazeutische Zusammensetzung, die
(a) Risperidon oder ein Salz oder Solvat davon,
(b) Polymethacrylat,
(c) Zuckeralkohol und
(d) Sprengmittel enthält, welches (i) vernetztes Polyvinylpyrrolidon und (ii) niedrig substituierte Hydroxypropylcellulose enthält.

2. Zusammensetzung nach Anspruch 1, bei der das Polymethacrylat (b) ein Polymer oder Copolymer auf Basis von mindestens einem von Dimethylaminoethylmethacrylaten, Methacrylsäure und Methacrylsäureestern ist.

3. Zusammensetzung nach Anspruch 1 oder 2, bei der das Polymethacrylat (b) ein Methacrylsäurecopolymer oder ein Copolymer von Dimethylaminoethylmethacrylaten und Methacrylsäureestern ist.

4. Zusammensetzung nach einem der Ansprüche 1 bis 3, bei der der Zuckeralkohol (c) ausgewählt ist aus Sorbit oder Mannit.

5. Zusammensetzung nach einem der Ansprüche 1 bis 4, bei der die niedrig substituierte Hydroxypropylcellulose einen Gehalt an Hydroxypropylgruppen von 7,0 bis 12,9 Gew.% aufweist.

6. Zusammensetzung nach einem der Ansprüche 1 bis 5, bei der das Sprengmittel (d) auch zusätzliches Sprengmittel ausgewählt aus (iii) Calciumsilikat, Natriumstärkeglykolat oder Croscarmellose-Natrium enthält.

7. Zusammensetzung nach einem der Ansprüche 1 bis 6, die
(a) 0,05 bis 4,0 Gew.% Risperidon oder ein Salz oder Solvat davon enthält.

8. Zusammensetzung nach einem der Ansprüche 1 bis 7, die
(b) 0,1 bis 20,0 Gew.-% Polymethacrylat enthält.

9. Zusammensetzung nach einem der Ansprüche 1 bis 8, die
(C) 30,0 bis 90,0 Gew.-% Zuckeralkohol enthält.

10. Zusammensetzung nach einem der Ansprüche 1 bis 9, die
(d) 5,0 bis 50,0 Gew.-% Sprengmittel enthält.

11. Zusammensetzung nach einem der Ansprüche 1 bis 10, die
(d) (i) 1,0 bis 30,0 Gew.% vernetztes Polyvinylpyrrolidon enthält.

12. Zusammensetzung nach einem der Ansprüche 1 bis 11, die
(d) (ii) 1,0 bis 20,0 Gew.% niedrig substituierte Hydroxypropylcellulose enthält.

13. Zusammensetzung nach einem der Ansprüche 1 bis 12, die in Form einer Tablette vorliegt.

14. Zusammensetzung nach einem der Ansprüche 1 bis 13, bei der das Gewichtsverhältnis von (b) zu (a) mindestens 3 zu 1 beträgt.

15. Verfahren zur Herstellung der Zusammensetzung nach einem der Ansprüche 1 bis 14, bei dem
(i) Körnchen hergestellt werden, die Risperidon oder ein Salz oder Solvat davon (a) und Polymethacrylat (b) enthalten, und
(ii) die Körnchen mit Zuckeralkohol (c) und Sprengmittel (d) gemischt werden, und
(iii) gegebenenfalls die erhaltene Mischung zu der gewünschten Form geformt wird.

16. Verfahren nach Anspruch 15, bei dem die Körnchen in Stufe (i) auch einen Teil des Zuckeralkohols (c) enthalten.

## Revendications

1. Composition pharmaceutique à désintégration orale, qui comprend :
a) de la rispéridone, ou un sel ou un solvat de ce composé,
b) un polyméthacrylate,
c) un alcool de sucre,
d) et un agent désintégrant, comprenant :
i) de la poly(vinyl-pyrrolidone) réticulée,
ii) et de l'hydroxypropyl-cellulose à faible degré de substitution.

2. Composition conforme à la revendication 1, dans laquelle le polyméthacrylate (b) est un polymère ou copolymère à base d'au moins l'un du méthacrylate de diméthylaminoéthyle, de l'acide méthacrylique et des esters de l'acide méthacrylique.

3. Composition conforme à la revendication 1 ou 2, dans laquelle le polyméthacrylate (b) est un copolymère de l'acide méthacrylique ou un copolymère du méthacrylate de diméthylaminoéthyle et d'un ester de l'acide méthacrylique.

4. Composition conforme à l'une des revendications 1 à 3, dans laquelle l'alcool de sucre (c) est choisi parmi du sorbitol et du mannitol.

5. Composition conforme à l'une des revendications 1 à 4, dans laquelle l'hydroxypropyl-cellulose à faible degré de substitution contient des groupes hydroxypropyle à raison de 7,0 à 12,9 % en poids.

6. Composition conforme à l'une des revendications 1 à 5, dans laquelle l'agent désintégrant (d) comprend aussi un désintégrant supplémentaire (iii) choisi parmi du silicate de calcium, du glycolate d'amidon sodique et de la croscarmellose sodique.

7. Composition conforme à l'une des revendications 1 à 6, qui comprend :
a) de 0,05 à 4,0 % en poids de rispéridone ou d'un sel ou d'un solvat de ce composé.

8. Composition conforme à l'une des revendications 1 à 7, qui comprend
b) de 0,1 à 20,0 % en poids de polyméthacrylate.

9. Composition conforme à l'une des revendications 1 à 8, qui comprend
c) de 30,0 à 90,0 % en poids d'alcool de sucre.

10. Composition conforme à l'une des revendications 1 à 9, qui comprend
d) de 5,0 à 50,0 % en poids d'agent désintégrant.

11. Composition conforme à l'une des revendications 1 à 10, qui comprend
d) i) de 1,0 à 30,0 % en poids de poly(vinyl-pyrrolidone) réticulée.

12. Composition conforme à l'une des revendications 1 à 11, qui comprend
d) ii) de 1,0 à 20,0 % en poids d'hydroxypropyl-cellulose à faible degré de substitution.

13. Composition conforme à l'une des revendications 1 à 12, qui se présente sous forme de comprimé.

14. Composition conforme à l'une des revendications 1 à 13, dans laquelle le rapport pondéral du composant (b) au composant (a) vaut au moins 3/1.

15. Procédé de préparation d'une composition conforme à l'une des revendications 1 à 14, comportant les étapes suivantes :
i) préparer des granules contenant de la rispéridone ou un sel ou solvat de ce composé (a) et un polyméthacrylate (b),
ii) et mélanger ces granules avec l'alcool de sucre (c) et l'agent désintégrant (d),
iii) et en option, mouler le mélange ainsi obtenu en objets de la forme voulue.

16. Procédé conforme à la revendication 15, dans lequel les granules préparés dans l'étape (i) contiennent aussi une partie de l'alcool de sucre (c).
